# EUROPEAN PATENT APPLICATION

(11) **EP 4 748 849 A1**
(43) Date of publication of application: **27.05.2026**
(21) Application number: 25816126.4
(22) Date of filing: 20.02.2025
(51) Int. Cl.: C07K 14/34, C12N 15/77, C12P 13/10

(54) **NOVEL PROMOTER AND METHOD FOR PRODUCING L-ARGININE USING SAME**

(30) Priority: 31.05.2024 KR 20240071881; 07.10.2024 KR 20240135801
(71) Applicant: CJ Cheiljedang Corporation, Seoul 04560 (KR)
(72) Inventor: KIM, Gyuree, Seoul 04560 (KR); KIM, Ye-Eun, Seoul 04560 (KR); JUNG, Hwi-Min, Seoul 04560 (KR); KIM, Bina, Seoul 04560 (KR); HWANG, Yeji, Seoul 04560 (KR); KIM, Eunji, Seoul 04560 (KR); CHOI, Jin-Geun, Seoul 04560 (KR); SHIM, Jihyun, Seoul 04560 (KR); RHO, Jin Ah, Seoul 04560 (KR)
(74) Representative: Meissner Bolte Partnerschaft mbB
(86) International application number: PCT/KR2025/002415
(87) International publication number: WO 2025/249708

(57) **Abstract**

The present disclosure relates to a novel polynucleotide having promoter activity and a method for producing L-arginine using same. A microorganism which has, introduced thereinto, the novel polynucleotide of the present disclosure having a mutation at a specific position in the promoter region of the BBD29_14250 gene exhibits a markedly increased ability to produce L-arginine, and thus the novel polynucleotide can be usefully employed for efficient production of L-arginine.

## Description

### [TECHNICAL FIELD]

### Cross-reference to related application(s)

The present disclosure claims the benefit of the priority based on Korean Patent Application No. 10-2024-0071881 filed on May 31, 2024, and Korean Patent Application No. 10-2024-0135801 filed on October 7, 2024, and the entire contents disclosed in the documents of the corresponding Korean patent applications are incorporated as a part of the present disclosure.

The present disclosure relates to a novel promoter and a method for producing L-arginine using the same, and more particularly, to a novel polynucleotide having a promoter activity, a vector comprising the polynucleotide, a host cell transformed with the vector, and a method for producing L-arginine using the host cell.

### [BACKGROUND ART]

Efforts such as genetic manipulation and/or introduction of foreign genes in a biosynthetic pathway have been continued in order to produce a target substance such as an amino acid or a useful substance, which can be used for various purposes such as feed, pharmaceuticals, and food, at a high titer using a microorganism. As one of such methods, there is a method of inducing overexpression of a target gene in a microorganism, and for this, a gene expression system with high-efficiency is required. Since the promoter is one of the factors that greatly affects the level of gene expression and expression regulation, the development of a useful promoter is essential for developing an expression system.

Microorganisms of the genus *Corynebacterium* are microorganisms that produce amino acids including L-arginine, and unlike other industrial microorganisms such as *Escherichia coli* or *Bacillus subtilis,* the general structure of promoter sequences for gene expression in microorganisms of the genus *Corynebacterium* has not been known. Therefore, promoters have been developed by removing the promoter part of an antibiotic resistance gene such as chloramphenicol, introducing chromosomal DNA isolated from a microorganism of the genus *Corynebacterium* thereinto after cleaving the same with appropriate restriction enzymes, and then measuring the antibiotic resistance of a strain obtained by transforming a microorganism of the genus *Corynebacterium* with this. As strong promoters derived from microorganisms of the genus *Corynebacterium,* Pcj1~7 promoters of various strengths are known (US 7662943 B2).

With the increase in demand for L-arginine, there is a continued need for the development of strong promoters for the overexpression of L-arginine-related genes as a method for producing L-arginine with high yield in microorganisms belonging to the genus *Corynebacterium.*

### [DISCLOSURE]

### [TECHNICAL PROBLEM]

One object of the present disclosure is to provide a novel polynucleotide.

Another aspect of the present disclosure is to provide an expression cassette comprising the polynucleotide and a target gene.

Other aspect of the present disclosure is to provide a microorganism comprising the polynucleotide; or the polynucleotide and a target gene operably linked thereto.

Other aspect of the present disclosure is to provide a method of producing L-arginine, comprising culturing the microorganism in a medium.

Other aspect of the present disclosure is to provide a use of the microorganism for producing L-arginine.

### [TECHNICAL SOLUTION]

Description thereof in detail is as follows. Meanwhile, each description and embodiment disclosed in the present disclosure may also be applied to other descriptions and embodiments. That is, all combinations of various elements disclosed in the present disclosure fall within the scope of the present disclosure. In addition, it cannot be considered that the scope of the present disclosure is limited by the specific descriptions described below. Furthermore, those skilled in the art will recognize, or be able to ascertain using no more than routine experimentation, many equivalents to the specific aspects of the present disclosure described in the present disclosure. In addition, such equivalents are intended to be included in the present disclosure.

Furthermore, those skilled in the art can recognize or confirm numerous equivalents to the specific aspects of the present application disclosed in the present application using only common experiments. In addition, these equivalents are intended to be included in the present application.

One aspect of the present disclosure provides a polynucleotide comprising any one nucleotide sequence selected from the group consisting of SEQ ID NO: 15 to SEQ ID NO: 21. In one embodiment, the polynucleotide may be a polynucleotide comprising the nucleotide sequence of SEQ ID NO: 21.

In the present disclosure, the phrase "a polynucleotide or polypeptide comprises a specific nucleotide sequence (nucleic acid sequence, base sequence) or amino acid sequence" may mean that the polynucleotide or polypeptide consists of or essentially comprises the specific nucleotide sequence (nucleic acid sequence, base sequence) or amino acid sequence.

In the present disclosure, the term "polynucleotide" may refer to one in which 2 or more, 5 or more, 10 or more, 13 or more, 20 or more, or 30 or more nucleotide monomers are included and the nucleotide monomers are covalently linked to form a chain form.

The polynucleotide of the present disclosure may have promoter activity and /or may be used as a universal promoter.

In one embodiment, the polynucleotide may have promoter activity for the expression in microorganisms of the genus *Corynebacterium.*

The polynucleotide having promoter activity may be used interchangeably with "mutant promoter" in the present disclosure, and all of the terms described above may be used in this disclosure.

The polynucleotide according to one embodiment of the present disclosure may be utilized as a synthetic promoter having strong activity of inducing expression.

In the present disclosure, the term "promoter" may refer to a DNA region that include a binding site for a polymerase and initiates transcription of a target gene at its downstream. The promoter may be located at 5'-terminus direction of a transcription start site. The promoter may be operably and/or controllably (enhancing or weakening expression) linked upstream (5'-terminus direction) of the target gene. For example, the promoter may be linked in a forward direction on the upstream side of the target gene to enhance (increase) the expression of the gene, or may be linked in a reverse direction on the downstream side of the target gene to weaken (decrease) the expression of the gene. When a promoter is introduced in a reverse direction to downstream of a target gene, for example, downstream of a stop codon, preferably between the stop codon and the top of a transcription terminator, it may weaken the expression of the gene by inducing transcription in the direction opposite to the normal transcription direction of the corresponding gene, thereby causing an RNA polymerase complex to collide with an RNA polymerase complex of the normal direction during the transcription process.

The polymerase, also called RNA polymerase or DNA-dependent RNA polymerase, may refer to an enzyme that synthesizes a primary transcript RNA from DNA. The polymerase may be an RNA polymerase of a prokaryotic cell or an RNA polymerase of a eukaryotic cell (for example, RNA polymerase I, RNA polymerase II, RNA polymerase III, RNA polymerase IV, or RNA polymerase V, etc.).

The polynucleotide according to one embodiment may be natural or non-natural, and for example, may be non-natural one which is synthesized chemically or recombinantly.

In the present disclosure, the phrase "a polynucleotide or polypeptide comprises a specific nucleotide sequence (nucleic acid sequence, base sequence) or amino acid sequence" may mean that the polynucleotide or polypeptide consists of or consists essentially of the specific nucleotide sequence (nucleic acid sequence, base sequence) or amino acid sequence, and may be interpreted as including (or not excluding) a sequence in which a mutation (deletion, substitution, modification, and/or addition) is added to the specific nucleic acid sequence (base sequence) or amino acid sequence within the scope of maintaining the original function and/or desired function of the polynucleotide or polypeptide. In addition, the expression "consists of a nucleotide sequence" does not exclude cases in which addition, and/or deletion, and/or mutation of nucleotides may occur during the process of linking to the target gene, such as the use of a restriction enzyme, when the polynucleotide is used by linking to the target gene as a promoter.

In one example, a polynucleotide or polypeptide "comprising a specific nucleotide sequence (nucleic acid sequence, base sequence) or amino acid sequence" may mean that the polynucleotide or polypeptide (i) consists of or consist essentially of the specific nucleotide sequence (nucleic acid sequence, base sequence) or amino acid sequence, or (ii) consists of or consists essentially of a nucleotide sequence or amino acid sequence that has at least 60%, 70%, 80%, 85%, 90%, 91%, 92%, 93%, 94%, 95%, 96%, 97%, 98%, 98%, 99.5%, or 99.9% homology with the specific nucleotide sequence (nucleic acid sequence, base sequence) or amino acid sequence and maintains its original function and/or desired function.

In one embodiment, the polynucleotide of the present disclosure may comprise the nucleotide sequence of SEQ ID NO: 15, and the nucleotide sequence of SEQ ID NO: 15 may be a sequence in which a portion of the promoter sequence of the PBBD29_12015 gene is mutated.

In one embodiment, the polynucleotide of the present disclosure may comprise the nucleotide sequence of SEQ ID NO: 16, and the nucleotide sequence of SEQ ID NO: 16 may be a sequence in which a portion of the promoter sequence of the PBBD29_07560 gene is mutated.

In one embodiment, the polynucleotide of the present disclosure may comprise the nucleotide sequence of SEQ ID NO: 17, and the nucleotide sequence of SEQ ID NO: 17 may be a sequence in which a portion of the promoter sequence of the PBBD29_12275 gene is mutated.

In one embodiment, the polynucleotide of the present disclosure may comprise the nucleotide sequence of SEQ ID NO: 18, and the nucleotide sequence of SEQ ID NO: 18 may be a sequence in which a portion of the promoter sequence of the PBBD29_01740 gene is mutated.

In one embodiment, the polynucleotide of the present disclosure may comprise the nucleotide sequence of SEQ ID NO: 19, and the nucleotide sequence of SEQ ID NO: 19 may be a sequence in which a portion of the promoter sequence of the PBBD29_04965 gene is mutated.

In one embodiment, the polynucleotide of the present disclosure may comprise the nucleotide sequence of SEQ ID NO: 20, and the nucleotide sequence of SEQ ID NO: 20 may be a sequence in which a portion of the promoter sequence of the PBBD29_05235 gene is mutated.

In one embodiment, the polynucleotide of the present disclosure may comprise a nucleotide sequence of SEQ ID NO: 21, and the nucleotide sequence of SEQ ID NO: 21 may be a sequence in which a portion of the promoter sequence of the PBBD29_14250 gene is mutated.

In the present disclosure, the term "variation" refers to a genetically or non-genetically stable phenotypic change, and may be used interchangeably with "mutation" in the present disclosure.

The polynucleotide (mutant promoter) of the present disclosure may have changed (increased or decreased) promoter activity compared to a polynucleotide that does not comprise a variation (a wild-type or pre-variation polynucleotide). The polynucleotide may regulate (increase or decrease) the expression of a target gene operably linked thereto, or the expression or activity of a protein encoded by the target gene, and furthermore, may regulate the expression of other genes besides the target gene.

The "target gene" means a gene for which expression is intended to be regulated by the polynucleotide of the present disclosure, and in the case of a gene encoding a protein, may be used interchangeably with 'a gene encoding a target protein'. A protein encoded by the target gene may be expressed as a "target protein", and a gene encoding the "target protein" may be expressed as a "target gene".

The amino acid coding sequence of the target gene, may be variously modified within a range that does not change the protein sequence encoded by the target gene, due to the degeneracy of the codon or in consideration of a preferred codon (codon usage frequency) in an organism in which the target gene is to be expressed.

The polynucleotide of the present disclosure, when introduced into a suitable host cell together with a target gene operably linked thereto, may have an activity of increasing the production ability (production amount) of a target substance of the host cell, for example, the production ability (production amount) of an amino acid.

In one embodiment, the polynucleotide may be for increasing amino acid production ability (production amount), and specifically, may be for increasing the production ability (production amount) of L-arginine.

In addition, the nucleotide sequence of the polynucleotide may be further modified by conventionally known mutagenesis methods, for example, directed evolution and site-directed mutagenesis, and the like, to the extent that it maintains a corresponding biological activity (promoter activity) and/or a desired activity (e.g., an activity of increasing production of a target substance in a host cell).

Accordingly, the polynucleotide of the present disclosure may comprise a nucleotide sequence having at least 70%, 80%, 85%, 90%, 95%, 96%, 97%, 98%, or 99% or more, but less than 100% of homology or identity with one nucleotide sequence selected from the group consisting of SEQ ID NO: 15 to SEQ ID NO: 21, or may comprises a sequence having the homology or identity but having some sequences added, deleted, or modified.

In the present disclosure, the polynucleotide may essentially consist of any one nucleotide sequence selected from the group consisting of SEQ ID NO: 15 to SEQ ID NO: 21. In another embodiment, the polynucleotide of the present disclosure may consist of any one nucleotide sequence selected from the group consisting of SEQ ID NO: 15 to SEQ ID NO: 21.

In the present disclosure, the term, 'homology' or 'identity' means the degree of similarity between two given amino acid sequences or nucleotide sequences and may be expressed as a percentage. The terms homology and identity may often be used interchangeably.

The sequence homology or identity of a conserved polynucleotide or polypeptide is determined by a standard alignment algorithm, and default gap penalties established by the program used may be employed together. Substantially, homologous or identical sequences can generally hybridize with the entire sequence or a portion thereof under medium or high stringent conditions. It is apparent that hybridization also includes hybridization with a polynucleotide containing a general codon or codon by considering the codon degeneracy in the polynucleotide.

Whether any two polynucleotide or polypeptide sequences have homology or identity may be determined, for example, by using a known computer algorithm such as the "FASTA" program using default parameters as in Pearson et al (1988) [Proc.Natl. Acad. Sci. USA 85]: 2444. Alternatively, the Needleman-Wunsch algorithm (Needleman and Wunsch, 1970, J. Mol. Biol. 48: 443-453), as performed in the Needleman program of the EMBOSS package (EMBOSS: The European Molecular Biology Open Software Suite, Rice et al., 2000, Trends Genet. 16: 276-277) (version 5.0.0 or a later version), may be used for the determination (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073)). For example, homology or identity may be determined using BLAST of the National Center for Biotechnology Information (NCBI) database, or ClustalW.

The homology or identity of a polynucleotide or a polypeptide may be determined by comparing sequence information using a GAP computer program such as, for example, Needleman et al. (1970), J Mol Biol. 48:443, as known in, for example, Smith and Waterman, Adv. Appl. Math (1981) 2:482. In summary, the GAP program can be defined as the value obtained by dividing the number of similarly aligned symbols (i.e., nucleotides or amino acids) by the total number of symbols in the shorter of the two sequences. Default parameters for the GAP program may include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or a gap opening penalty of 10, a gap extension penalty of 0.5); and (3) no penalty for end gaps.

In one example, a polynucleotide comprising a specific nucleotide sequence provided in the present disclosure may be interpreted as comprising not only the specific nucleotide sequence or a nucleotide sequence substantially equivalent thereto, but also a polynucleotide fragment comprising a nucleotide sequence complementary to the specific nucleotide sequence. Specifically, the polynucleotide having the complementarity can be identified under the conditions described below: these conditions are specifically described in known literature. For example, conditions under which genes having high complementarity of 60% or more, 70% or more, 80% or more, 85% or more, 90% or more, 91% or more, 92% or more, 93% or more, 94% or more, 95% or more, 96% or more, 97% or more, 98% or more, 98% or more, 99.5% or more, or 99.9% or more, hybridize with each other, and the genes having lower complementarity do not hybridize, or conditions of washing once, specifically two to three times, at a salt concentration and temperature corresponding to the washing conditions of a conventional Southern hybridization, i.e., 60°C, 1x SSC (saline-sodium citrate buffer), and 0.1% (w/v) SDS (Sodium Dodecyl Sulfate); 60°C, 0.1x SSC, and 0.1% SDS(Sodium Dodecyl Sulfate); or 68°C, 0.1x SSC, and 0.1% SDS, may be listed, but are not limited thereto. Hybridization requires that two nucleotides have complementary sequences, but some base mismatches may be allowed depending on the stringency of hybridization. The term "complementary" may be used to describe the relationship between nucleotide that can hybridize with each other. For example, in the case of DNA, adenosine is complementary to thymine and cytosine is complementary to guanine. The stringency of hybridization between polynucleotides depends on the length and degree of complementarity of the polynucleotides, which is well known in the relevant art (see Sambrook et al., supra, 9.50-9.51, 11.7-11.8).

Furthermore, the polynucleotide of the present disclosure may be operably linked to a gene encoding a target protein, i.e., a target gene.

In the present disclosure, the term "operably linked" means that a polynucleotide having promoter activity of the present disclosure is functionally linked to the gene sequence so as to initiate and mediate transcription of the target gene. An operable linkage can be prepared using known gene recombination techniques in the art, and site-specific DNA cleavage and ligation can be produced using cleavage and ligation enzymes and the like in the art, but are not limited thereto.

When the polynucleotide is operably linked to the target gene, some nucleotides may be added, deleted, and/or mutated for the use of the cleavage and ligation enzymes and the like.

In one embodiment, the target gene may be a gene encoding a protein involved in the production of L-arginine of the present disclosure, but is not limited thereto. The protein involved in the production of L-arginine may be a protein involved in at least one process or step of an intracellular production pathway (e.g., biosynthesis, metabolism, bioconversion, etc.), intracellular transport, and/or an extracellular excretion pathway of L-arginine, and may be selected from the group consisting of, for example, an enzyme (various synthases, lyases, kinases, carboxylases (e.g., pyruvate carboxylase, etc.), reductases, oxidases, decarboxylases, dehydrogenases, dehydratases, transferases, epimerases, etc.), an intermediate, a transport protein, a membrane protein (channel, etc.), and the like, but is not limited thereto.

In one embodiment, the target gene may be, but is not limited to, the PBBD29_12015, PBBD29_07560, PBBD29_12275, PBBD29_01740, PBBD29_04965, PBBD29_05235, or PBBD29_14250 gene.

Another aspect of the present disclosure provides an expression cassette comprising a polynucleotide of the present disclosure and a target gene.

The polynucleotide and target gene are as described above.

In the present disclosure, the term, "expression cassette," refers to a unit cassette that comprises a promoter and a target gene operably linked thereto, and is capable of expressing the target gene at downstream of the promoter. Inside or outside such a gene expression cassette, various factors that can aid in the efficient expression of the target gene may be included. The gene expression cassette, in addition to the promoter operably linked to the target gene, may typically comprise a transcription termination signal, a ribosome binding site, and a translation termination signal, but is not limited thereto.

Another aspect of the present disclosure provides a vector comprising: the polynucleotide of the present disclosure; the polynucleotide and a target gene operably linked thereto; or the expression cassette.

The polynucleotide, the target gene, and the expression cassette are as described above. The vector may comprise a target gene operably linked to the polynucleotide.

In the present disclosure, the term "vector" refers to a DNA preparation that comprises the base sequence of the polynucleotide encoding the target protein, operably linked to a suitable regulatory sequence, so as to be able to express the target protein in a suitable host. The regulatory sequence may comprise a promoter capable of initiating transcription, any operator sequence for regulating transcription, a sequence encoding a suitable mRNA ribosome binding site, and/or a sequence that regulates the termination of transcription and/or translation. After being transformed into a suitable host cell, a vector may be expressed independently of the genome of the host cell or may be integrated into the genome of the host cell.

A vector usable in the present disclosure is not particularly limited as long as it is replicable in a host cell, and may be selected from among all commonly used vectors. Examples of commonly used vectors include plasmids, cosmids, viruses, bacteriophages, and the like, in a natural or recombinant state. For example, as the vector, pWE15, M13, MBL3, MBL4, IXII, ASHII, APII, t10, t11, Charon4A, and Charon21A, and the like may be used as phage vectors or cosmid vectors, and pDZ series, pBR series, pUC series, pBluescriptII series, pGEM series, pTZ series, pCL series, and pET series, and the like may be used as plasmid vectors. Specifically, examples may include, but are not limited to, pDZ, pDC, pACYC177, pACYC184, pCL, pECCG117, pUC19, pBR322, pMW118, pCC1BAC, pDCM2, pDC24 vectors, and the like.

A vector usable in the present disclosure may be an expression vector or an insertion vector for insertion into a host cell chromosome. The insertion of the target DNA into the host cell chromosome using an insertion vector may be performed by any method known in the art, for example, by homologous recombination or a CRISPR system, but is not limited thereto. The vector may further include a selection marker for confirming whether the vector has been transformed or further whether the target DNA has been inserted into the chromosome. The selection marker may be selected and used from among genes that confer a selectable phenotype, such as drug resistance, auxotrophy, resistance to cytotoxic agents, or expression of surface proteins. In an environment treated with a selective agent, only cells expressing the selection marker can survive or exhibit a different phenotype, and thus, transformed cells can be selected.

In the present disclosure, the term "transformation" refers to introducing a target polynucleotide into a host cell. A transformed polynucleotide may be inserted into the chromosome of the host cell or may be located extrachromosomally. Furthermore, the polynucleotide may be DNA and/or RNA, and it does not matter in what form it is introduced, as long as it can be introduced into a host cell and function therein. For example, the polynucleotide may be introduced into a host cell in the form of an expression cassette, which is a gene construct comprising all elements necessary for its own expression, or in the form of a vector comprising the same.

The transformation method comprises any method of introducing the target polynucleotide into a cell, and may be performed by selecting a suitable standard technique as is known in the art, depending on the host cell. For example, there are electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method, and the like, but it is not limited thereto.

Another aspect of the present disclosure provides a microorganism (host cell) comprising the polynucleotide of the present disclosure, the polynucleotide and a target gene operably linked thereto, or the expression cassette.

The polynucleotide, the target gene, or the expression cassette is as described above.

The polynucleotide, the polynucleotide and the target gene operably linked thereto, or the expression cassette may be introduced into a microorganism by transformation, but is not limited thereto.

In the present disclosure, the term "microorganism" includes both a wild-type microorganism and a microorganism that has been naturally or artificially genetically modified, and also includes a microorganism in which a specific mechanism is attenuated or enhanced due to causes such as the insertion of an exogenous gene or the enhancement or attenuation of the activity of an endogenous gene.

The microorganism may be a microorganism that naturally expresses a target gene or a microorganism that has a production ability of target product, or may be a microorganism in which an expression ability of target gene or a production ability of target product is conferred to a parent strain that does not naturally express a target gene or does not have a production ability of target product, but is not limited thereto. In one embodiment, the microorganism may be a microorganism that naturally produces an amino acid, specifically a microorganism that produces L-arginine.

In the present disclosure, the term "target product" refers to a biologically active substance that is intended to be produced or whose production is intended to be regulated (increased or decreased) by using the polynucleotide provided in the present disclosure, the polynucleotide and a target gene operably linked thereto, an expression cassette comprising the polynucleotide and the target gene, a vector comprising the same, and/or a microorganism comprising the same, and is a concept that includes not only the biologically active substance to be finally produced but also a target protein that can be produced by the microorganism. For example, it may refer to the target protein itself encoded by the target gene, and/or any biologically active substance produced through the involvement of the target protein. The biologically active substance refers to any substance that is produced or derived from an organism (e.g., a cell) or has a predetermined function in vivo or within a cell, and may be, for example, an amino acid (glycine, alanine, valine, arginine, isoleucine, threonine, serine, cysteine, glutamine, methionine, aspartic acid, asparagine, glutamic acid, lysine, arginine, histidine, phenylalanine, tyrosine, tryptophan, proline, O-acetyl homoserine, etc.), a nucleic acid, a vitamin (vitamin A, B (B1, B2, B3, B5, B6, B7, B9, B12, etc.), C, D, E, K, etc.), a protein (the target protein or a protein other than it, for example, a hormone, a growth factor, a cytokine, an immunoglobulin (antibody), an antigen protein, a receptor, a ligand, a functional fragment thereof (a fragment retaining a desired function), a fusion protein in which two or more are fused, etc.), a sugar (e.g., a monosaccharide, a disaccharide, a polysaccharide, a sugar alcohol, etc.), a fatty acid (myristoleic acid, palmitoleic acid, sapienic acid, oleic acid, elaidic acid, vaccenic acid, linoleic acid, linoelaidic acid, arachidonic acid, eicosapentaenoic acid (EPA), erucic acid, docosahexaenoic acid (DHA), etc.), an organic acid (lactic acid, citric acid, oxalic acid, uric acid, butyric acid, stearic acid, propionic acid, etc.), and the like, but is not limited thereto. In addition, if it is a substance produced through the involvement of the target protein, metabolites thereof (polyhydroxyalkanoate (PHA), etc.), precursors thereof, derivatives thereof that maintain their biological activity, and the like may also be included in the target product , in addition to the substances described above.

In one embodiment, the microorganism comprising the polynucleotide of the present disclosure and a target gene operably linked thereto may be a microorganism with an increased production ability of an amino acid as a target product, and specifically may be a microorganism with an increased production ability of L-arginine. The microorganism with the increased production ability of L-arginine may be a microorganism with an increased production ability of L-arginine compared to a microorganism that does not comprise the polynucleotide of the present disclosure, for example, a microorganism in which the expression of at least one gene selected from the group consisting of BBD29_12015, BBD29_07560, BBD29_12275, BBD29_01740, BBD29_04965, BBD29_05235, and BBD29_14250 is regulated by a polynucleotide before the introduction of a mutation, but is not limited thereto. The polynucleotide before the introduction of the mutation may be a wild-type polynucleotide, and specifically may consist of the nucleotide sequence selected from the group consisting of SEQ ID NO: 22 to SEQ ID NO: 28.

In the present disclosure, a "non-modified microorganism" does not exclude a strain including a naturally occurring mutation, and may refer to a wild-type strain or a naturally-occurring strain itself, or a strain before its properties are changed by genetic variation due to natural or artificial factors. For example, the non-modified microorganism may refer to a strain in which a mutant polynucleotide has not been introduced into the promoter region of at least one gene selected from the group consisting of BBD29_12015, BBD29_07560, BBD29_12275, BBD29_01740, BBD29_04965, BBD29_05235, and BBD29_14250 described in the present disclosure, or a strain before such introduction. The "non-modified microorganism" may be used interchangeably with "pre-modification strain", "pre-modification microorganism", "non-mutant strain", " non-modified strain", "non-mutant microorganism", or "reference microorganism".

The microorganism of the present disclosure can include, without limitation, any microorganism into which the polynucleotide of the present disclosure can be introduced and function as a promoter.

In one embodiment, the microorganism may be a microorganism of *Corynebacterium* sp., a microorganism of an *Escherichia* sp., or a microorganism of *Bacillus* sp., but is not limited thereto.

Specifically, the microorganism may be a microorganism of the genus *Corynebacterium,* and more specifically, may include *Corynebacterium glutamicum, Corynebacterium stationis, Corynebacterium thermoaminogenes, Corynebacterium glutamicum, Brevibacterium flavum, Brevibacterium lactofermentum,* and strains prepared therefrom, but is not limited thereto. Specifically, the microorganism of the genus *Corynebacterium* may be *Corynebacterium glutamicum.*

In one embodiment, the microorganism may be *Corynebacterium glutamicum* in which a mutation has been introduced into the promoter of at least one gene selected from the group consisting of BBD29_12015, BBD29_07560, BBD29_12275, BBD29_01740, BBD29_04965, BBD29_05235, and BBD29_14250, but is not limited thereto.

In one embodiment, the microorganism of the present disclosure with increased L-arginine productivity (production amount) may have an L-arginine productivity (production amount) increased by about 1% or more, about 2% or more, about 3% or more, about 4% or more, about 5% or more, about 5.5% or more, or about 5.8% or more (the upper limit is not particularly limited, and may be, for example, about 200% or less, about 150% or less, about 100% or less, or about 90% or less) compared to a parent strain before mutation or an unmodified microorganism, but is not limited thereto. In another embodiment, the microorganism of the present disclosure with increased L-arginine productivity (production amount) may have an L-arginine productivity (production amount) increased by about 1.01 fold or more, about 1.02 old or more, about 1.03 fold or more, about 1.04 fold or more, about 1.05 fold or more, about 1.055 fold or more, or about 1.058 fold or more (the upper limit is not particularly limited, and may be, for example, about 10-fold or less, about 5-fold or less, about 3-fold or less, about 2-fold or less, or about 1.5-fold or less) compared to a parent strain before mutation or an non-modified microorganism, but is not limited thereto.

The term "about" is a range including all of ±0.5, ±0.4, ±0.3, ±0.2, ±0.1, etc., and includes all numerical values in a range equivalent or similar to the numerical value that follows the term about, but is not limited thereto.

Another aspect of the present disclosure provides a composition for producing L-arginine, comprising at least one selected from the group consisting of the polynucleotide of the present disclosure, the polynucleotide and a target gene operably linked thereto, the expression cassette, the vector, and the microorganism. The polynucleotide, the target gene, the expression cassette, the vector, the microorganism, and the like are as described above.

In one example, the composition for producing L-arginine may further comprise any suitable excipient conventionally used in a composition for producing L-arginine, and such an excipient may be, for example, a preservative, a wetting agent, a dispersing agent, a suspending agent, a buffer, a stabilizer, or an isotonic agent, and the like, but is not limited thereto.

Another aspect of the present disclosure provides a use of at least one selected from the group consisting of the polynucleotide of the present disclosure, the said polynucleotide and a target gene operably linked thereto, the expression cassette, the vector, and the microorganism, for producing L-arginine. The polynucleotide, the target gene, the expression cassette, the vector, the microorganism, and the like are as described above.

Another aspect of the present disclosure provides a use of at least one selected from the group consisting of the polynucleotide of the present disclosure, the said polynucleotide and a target gene operably linked thereto, the expression cassette, the vector, and the microorganism, for the preparation of a composition for producing L-arginine. The polynucleotide, the target gene, the expression cassette, the vector, the microorganism, and the like are as described above.

Another aspect of the present disclosure provides a method for producing a target product, the method comprising a step of culturing in a medium a microorganism comprising the polynucleotide of the present disclosure, the polynucleotide and a target gene operably linked thereto, the expression cassette or the vector.

The polynucleotide, the target gene, the expression cassette, the vector, the microorganism, and the target product are as described above.

In one embodiment, the target product may be an L-amino acid. Specifically, the target product may be L-arginine.

The method may further comprise a step of recovering the target product from the cultured microorganism, the culture, or both, after the step of culturing.

The term "culturing" in the present disclosure means growing a microorganism under appropriately and artificially controlled environmental conditions. The culturing process of the present disclosure may be performed according to a suitable medium and culture conditions known in the art. Such a culturing process can be easily adjusted and used by a person skilled in the art according to a selected strain. Specifically, the culturing may be a batch, continuous, and/or fed-batch culture, but is not limited thereto. These various methods are disclosed in, for example, "Biochemical Engineering" (James M. Lee, Prentice-Hall International Editions, pp138-176, 1991).

In the present disclosure, the term "medium" means a substance prepared by mixing nutrients required for culturing the microorganism of the present disclosure as main components, and supplies water, which is essential for survival and growth, as well as nutrients and growth factors. Specifically, as the medium and other culture conditions used for culturing the microorganism of the present disclosure, any medium used for culturing conventional microorganisms can be used without particular limitation, but the microorganism of the present disclosure can be cultured in a conventional medium containing a suitable carbon source, nitrogen source, phosphorus source, inorganic compound, amino acid and/or vitamin, while controlling temperature, pH, etc., under aerobic conditions. Specifically, a culture medium for a microorganism can be found in literature such as ["Manual of Methods for General Bacteriology" by the American Society for Bacteriology (Washington D.C., USA, 1981)].

The medium used for culturing must meet the requirements of a specific strain in an appropriate manner. For example, culturing can be performed in a conventional medium containing a suitable carbon source, nitrogen source, amino acid, vitamin, etc., while controlling temperature, pH, etc., under aerobic conditions. At this time, the carbon source includes carbohydrates such as glucose, fructose, and sucrose, and amino acids such as glutamic acid and cysteine. Specifically, natural organic nutrient sources such as starch hydrolysates and molasses can be used, and preferably carbohydrates such as glucose, fructose, sterilized pre-treated molasses (i.e., molasses converted to reducing sugars), and other appropriate amounts of carbon sources can be used in various ways without limitation, but are not limited thereto. As the nitrogen source, inorganic nitrogen sources such as ammonia; and amino acids such as glutamic acid and cysteine, and peptone, meat extract, yeast extract, etc., can be used as organic nitrogen sources. These nitrogen sources may be used alone or in combination, but are not limited thereto. In the medium, phosphoric acid, potassium dihydrogen phosphate or dipotassium hydrogen phosphate, or a corresponding sodium-containing salt may be used as a phosphorus source, but is not limited thereto. As the inorganic compound, magnesium sulfate, iron sulfate, manganese sulfate, and calcium chloride, etc., may be used, and in addition, amino acids, vitamins, and appropriate precursors, etc., may be included. These media or precursors may be added to the culture in a batch or continuous manner, but are not limited thereto.

During culturing, the pH of the culture can be adjusted by adding compounds such as potassium hydroxide, ammonia, and phosphoric acid to the culture in an appropriate manner. Furthermore, during culturing, an antifoaming agent such as a fatty acid polyglycol ester can be used to suppress foam generation. Furthermore, to maintain the aerobic state of the culture, oxygen or an oxygen-containing gas can be injected into the culture. The temperature of the culture is 27°C to 37°C, and specifically 30°C to 33°C. The culturing period can be continued until the desired production amount of the useful substance is obtained, and is specifically 20 to 160 hours.

In the present disclosure, the term "culture" means a substance comprising a medium in which a microorganism is growing or has completed growth under appropriately and artificially controlled environmental conditions. In a narrow sense, the culture does not include the grown microorganism, but it may be included in a broad sense. The "culture" may comprise various target substances discharged by the microorganism into the medium during its growth, along with the medium components formulated for the microorganism culture.

In the culturing of the present disclosure, the culture temperature may be maintained at 20°C to 45°C, specifically 25°C to 40°C, 25°C to 40°C, 25°C to 37°C, 25°C to 35°C, 27°C to 40°C, 27°C to 37°C, 27°C to 35°C, 30°C to 40°C, 30°C to 37°C, or 30°C to 35°C, and the culturing can be performed for about 10 to 160 hours, but is not limited thereto.

The target product produced by the culturing of the present disclosure may be secreted into the medium or may retain within the cells.

The step of recovering the target product may be to collect the target product using a suitable method known in the pertinent art, according to the culturing method, for example, a batch, continuous, or fed-batch culturing method. For example, various types of chromatography such as centrifugation, filtration, treatment with a crystallizing protein precipitating agent (salting out), extraction, ultrasonic disruption, ultrafiltration, dialysis, molecular sieve chromatography (gel filtration), adsorption chromatography, ion-exchange chromatography, and affinity chromatography, HPLC, or a combination of these methods may be used, but is not limited thereto, and the desired product of interest can be recovered from the medium or the microorganism using a suitable method known in the pertinent art.

Furthermore, the method for producing a target product of the present disclosure may additionally comprise a purification step. The purification can be performed using a suitable method known in the pertinent art. In one example, when the method for producing a target product of the present disclosure comprises both a recovery step and a purification step, the recovery step and the purification step may be performed at different times (or continuously) regardless of the order, or may be performed simultaneously or integrated into a single step, but is not limited thereto.

Another aspect of the present disclosure provides a method for increasing the productivity of a target product, the method comprising a step of culturing in a medium a microorganism comprising the polynucleotide of the present disclosure, the polynucleotide and a target gene, or an expression cassette comprising the polynucleotide and a target gene.

The polynucleotide, the target gene, the expression cassette, the microorganism, and the target product are as described above.

In one embodiment, the target product may be an L-amino acid, specifically L-arginine.

Another aspect of the present disclosure provides a method for preparing a microorganism with increased productivity of a target product, the method comprising a step of introducing into a microorganism, the polynucleotide of the present disclosure, the polynucleotide and a target gene, or an expression cassette comprising the polynucleotide and a target gene.

The polynucleotide, the target gene, the expression cassette, the microorganism, and the target product are as described above.

In one embodiment, the target product may be an L-amino acid, specifically L-arginine.

According to another aspect of the present disclosure, the present disclosure provides a composition, a method, a product, a process, or a use characterized by one or more elements disclosed in the present disclosure.

### [ADVANTAGEOUS EFFECTS]

The present disclosure relates to a novel polynucleotide having a promoter activity and a method for producing L-arginine using the same, and as the L-arginine productivity is significantly increased in a microorganism introduced with novel polynucleotide of the present disclosure, the novel polynucleotide can be usefully utilized for efficiently producing L-arginine.

### [MODE FOR INVENTION]

Hereinafter, the present disclosure will be described in more detail through examples. These examples are intended solely to illustrate the present disclosure more specifically, and it will be apparent to those skilled in the art that the scope of the present disclosure is not limited by these examples, in accordance with the gist of the present disclosure.

### EXAMPLE

(Throughout this specification, the term "%" used to indicate the concentration of a specific substance, unless otherwise specified, refers to (weight/weight) % for solid/solid, (weight/volume) % for solid/liquid, and (volume/volume) % for liquid/liquid.)

### EXAMPLE 1. Construction of an L-arginine-producing microorganisms

### EXAMPLE 1-1. Construction of Corynebacterium glutamicum CJR2 strain

To evaluate L-arginine production capacity, *Corynebacterium glutamicum* CJR2, in which *ΔargR* and *argB*(M54V) mutations were introduced into wild-type *Corynebacterium glutamicum* ATCC13869, was prepared (Ikeda, Masato et al., Applied and environmental microbiology 75(6)1635-41, 2009).

First, vectors for introducing the *argR* deletion and the *argB*(M54V) mutation were prepared. PCR using primer pairs of SEQ ID NOs: 1 and 2, and SEQ ID NOs: 3 and 4, and overlapping PCR using the primer pair of SEQ ID NOs: 1 and 4 were performed using the genomic DNA of *Corynebacterium glutamicum* ATCC13869 as a template to obtain a homologous recombination fragment having the *argR* deletion mutation sequence. To prepare a homologous recombination fragment having the *argB*(M54V) mutation in the same manner, PCR using primer pairs of SEQ ID NOs: 5 and 6, and SEQ ID NOs: 7 and 8, and overlapping PCR using SEQ ID NOs: 5 and 8 were performed. The PCR reaction was performed by denaturation at 95°C for 5 minutes; repeating denaturation at 95°C for 30 seconds, annealing at 60°C for 30 seconds, and polymerization at 72°C for 1 minute 27 times; followed by a polymerization reaction at 72°C for 5 minutes. After the fragments obtained above purification process, fusion cloning was performed using an In-Fusion^{®} HD cloning kit (Clontech) by cloning with the pDC24 vector (SEQ ID NO: 43) treated with SmaI restriction enzyme according to the manual, to obtain plasmids. The prepared vectors were named pDC24-ΔargR and pDC24-argB(M54V), respectively.

Subsequently, the *argR* deletion mutation was introduced into wild-type *Corynebacterium glutamicum* ATCC13869. Transformation was performed by electroporation using the prepared pDC24-Δ*argR* plasmid (van der Rest et al., Appl Microbiol Biotechnol 52:541-545, 1999). Then, secondary recombination was performed on a solid plate medium containing 4% sucrose, and PCR using the primer pair of SEQ ID NOs: 1 and 4 was performed on the transformants in which the secondary recombination was completed to confirm that the deletion mutation was introduced into the argR gene on the chromosome. At this time, the PCR reaction was performed under the same conditions as described above, and the transformant thus obtained was named CJR1.

### <Solid plate medium (pH 7.0)>

Glucose 10 g, Peptone 10 g, Beef extract 5 g, Yeast extract 5 g, Brain Heart Infusion 18.5 g, NaCl 2.5 g, Urea 2 g, Sorbitol 91 g, Agar 20 g (based on 1 liter of distilled water)

The *argB*(M54V) mutation was introduced into the *Corynebacterium glutamicum* CJR1 in the same manner as above. The prepared pDC24-*argB*(M54V) plasmid was used, and PCR using the primer pair of SEQ ID NOs: 5 and 8 was performed on the transformants in which the secondary recombination was completed to confirm that the M54V mutation was introduced into the argB gene on the chromosome, and the transformant was named CJR2.

The sequence information of the primers used in Example 1-1 is described in Table 1 below.

**[Table 1]**

| SEQ ID NO | Name | SEQUENCE(5'->3') |
|---|---|---|
| 1 | *argR*-5'-F | tgaattcgagctcggtaccccactggtgaactccttgtcc |
| 2 | *argR*-5'-R | ttgaactaggggcgctttaaaagttttccggtgttgacgg |
| 3 | *argR*-3'-F | ccgtcaacaccggaaaacttttaaagcgcccctagttcaa |
| 4 | *argR-3'-R* | gtcgactctagaggatcccccgttgaactgcttgccagcc |
| 5 | *argB-5'-F* | tgaattcgagctcggtaccctgcggctcgcacggttgctc |
| 6 | *argB*-5'-R | acggtgcgcaagaagaccacgtcggcagcaaaagcagcct |
| 7 | *argB*-3'-F | ggctgcttttgctgccgacgtggtcttcttgcgcaccgtg |
| 8 | *argB*-3'-R | gtcgactctagaggatccccctcttatcaggccaatcggt |

### EXAMPLE 1-2. Construction of Corynebacterium glutamicum CJR100 strain

Based on the CJR2 strain prepared in Example 1-1, a CJR100 strain in which the N-acetyl-gamma-glutamyl-phosphate reductase (hereinafter, *argC*) gene was enhanced was prepared.

In order to enhance the activity of *argC* (NCBI Accession No. BBD29_RS07530), which is an N-acetyl-gamma-glutamyl-phosphate reductase, a plasmid for enhancing *argC* activity was prepared by replacing the wild-type promoter of the *argC* gene with Po2 using the Po2 promoter (US 10273491 B2) known as a strong promoter. Upstream and downstream regions of the *argC* gene were obtained. Specifically, to prepare a strain into which *argC* with the Po2 promoter was introduced, PCR was performed to amplify the upstream region of the *argC* gene using primers of SEQ ID NO: 9 and SEQ ID NO: 10 and the gene fragment of the downstream region of the *argC* gene using primers of SEQ ID NO: 11 and SEQ ID NO: 12, respectively, using the chromosomal DNA of *Corynebacterium glutamicum* ATCC13869 as a template. In addition, a Po2 promoter fragment was obtained using SEQ ID NO: 13 and SEQ ID NO: 14 with the synthesized Po2 promoter as a template. As a polymerase for the PCR reaction, Pfu UltraTM High-Fidelity DNA Polymerase (Stratagene) was used, and PCR was performed in the same manner as in Example 1-1. As a result, an 86bp DNA fragment of the Po2 promoter region, a 610bp DNA fragment of the *Corynebacterium glutamicum* ATCC13869 *argC* upstream, and a 1086bp DNA fragment of the downstream were obtained, respectively. PCR was performed in the same manner as in Example 1-1 using the amplified promoter and DNA fragments as templates and using the primers of SEQ ID NO: 9 and SEQ ID NO: 12. After the two fragments obtained above were subjected to DNA purification, fusion cloning was performed by ligating them with a pDC24 plasmid treated with SmaI restriction enzyme using an In-Fusion^{®} HD cloning kit (Clontech). The resulting vector was named pDC24-Po2-argC.

Subsequently, the CJR2 strain prepared in Example 1-1 was transformed with the prepared pDC24-Po2-argC plasmid by electroporation (van der Rest et al., Appl Microbiol Biotechnol 52:541-545, 1999). Then, secondary recombination was performed on a solid plate medium containing 4% sucrose, and PCR was performed on the transformants for which the secondary recombination was completed using primers of SEQ ID NOs: 9 and 14 to confirm that the chromosomal *argC* gene was enhanced with the Po2 promoter. At this time, the PCR reaction was performed under the same conditions as described above, and the transformant thus obtained was named CJR100.

### <Solid plate medium (pH 7.0)>

10 g of glucose, 10 g of peptone, 5 g of beef extract, 5 g of yeast extract, 18.5 g of Brain Heart Infusion, 2.5 g of NaCl, 2 g of urea, 91 g of sorbitol, and 20 g of agar (based on 1 liter of distilled water)

The sequence information of the primers used in Example 1-2 is described in Table 2 below.

**[Table 2]**

| SEQ ID NO | Name | SEQUENCE(5'->3') |
|---|---|---|
| 9 | *argC*-5'-F | GTGAATTCGAGCTCGGTACCCGCCCCGAAAAGCCGTT AAAAG |
| 10 | *argC*-5'-R | tgccaaaattcacgattattgCTCGAGTCTAGAGACGGGTTA |
| 11 | *argC*-3'-F | ttattggaggagatcaaaacaATGACAATCAAGGTTGCAATC |
| 12 | *argC*-3'-R | CAGGTCGGCGTCGCACCTTAAGGGGATCCTCTAGAGT CGACC |
| 13 | Po2-F | caataatcgtgaattttggca |
| 14 | Po2-R | tgttttgatctcctccaataa |

### EXAMPLE 2: Selection of mutant strain with increased arginine productivity by artificial mutagenesis

### EXAMPLE 2-1: Random mutagenesis via UV irradiation

To select a mutant strain with increased arginine productivity, the arginine-producing strain of CJR100 prepared in Example 1, was spread on a nutrient medium containing agar and cultured at 30°C for 16 hours. The hundreds of colonies thus obtained were irradiated with UV (Ultraviolet mutation) at room temperature to induce random mutations in the genome of the strain.

### <Nutrient Medium (pH 7.2)>

Glucose 10g, meat extract 5g, polypeptone 10g, sodium chloride 2.5g, yeast extract 5g, agar 20g, urea 2g (per 1 liter of distilled water)

### EXAMPLE 2-2: Selection of strain with increased L-arginine productivity

To select a mutant strain with increased arginine productivity compared to the parent strain CJR100, CJR100 strain and the mutant strains in which random mutations were induced in Example 2-1, were cultured by the following method.

Each of the aforementioned strains was inoculated into a 96-Deep Well Plate-Dome (Bioneer) containing 400 µl of a seed medium, and cultured in a plate shaking incubator (TAITEC) at 32°C and 1200 rpm for about 48 hours. The arginine concentrations of about 3000 cultured strains were respectively checked via a near-infrared (NIR) spectroscopic analyzer, and the top 4 mutant strains with improved arginine productivity compared to the parent strain CJR100 were selected.

### <Seed Medium (pH 7.0)>

Glucose 20 g, peptone 10 g, yeast extract 5 g, urea 1.5 g, KH₂PO₄ 4 g, K₂HPO₄ 8g, MgSO₄·7H₂O 0.5 g, biotin 100 µg, thiamine HCl 1000 µg, calcium pantothenate 2000 µg, nicotinamide 2000 µg (based on 1 liter of distilled water)

To finally select strains with reproducibly increased L-arginine productivity from the 4 selected mutant strains, they were cultured and evaluated by the following method.

The control stain and the aforementioned four mutant strains were inoculated respectively into a 250 mL corner-baffle flask containing 25 mL of an arginine production medium, and then cultured with shaking at 32°C for 20 hours at 200 rpm. Then, 1 ml of a seed culture was inoculated into a 250 ml corner-baffled flask containing 24 ml of a production medium, and shake-cultured at 30°C and 200 rpm for 54 hours.

### <Seed Medium (pH 7.0)>

Glucose 20 g, peptone 10 g, yeast extract 5 g, urea 1.5 g, KH₂PO₄ 4 g, K₂HPO₄ 8 g, MgSO₄·7H₂O 0.5 g, biotin 100 ug, thiamine HCl 1000 ug, calcium-pantothenate 2000 ug, nicotinamide 2000 ug (based on 1 liter of distilled water)

### <Production medium (pH 7.2)>

5% glucose, 3% ammonium sulfate, 0.1% potassium phosphate monobasic, 0.2% magnesium sulfate heptahydrate, 1.5% CSL (corn steep liquor), 1% NaCl, 0.5% yeast extract, biotin 100 mg/L

After the cultivation was completed, the L-arginine concentration in the culture broth was analyzed using high-performance liquid chromatography (HPLC), and the L-arginine production concentrations of each mutant strain are shown in Table 3 below.

**[Table 3]**

| Strain name | L-Arg (g/L) |
|---|---|
| CJR100 | 5.8 |
| CJR100_mt1 | 6.0 |
| CJR100_mt2 | 2.5 |
| CJR100_mt3 | 6.3 |
| CJR100_mt4 | 5.9 |

As shown in Table 3 above, among the four selected mutant strains, CJR100_mt3 was finally selected as the mutant strain with the greatest increase in L-arginine production.

### EXAMPLE 2: Identification of mutations by whole-genome sequencing (WGS)

Whole-genome sequencing (WGS) was performed for CJR1000_mt3 selected in Example 2-2 to analyze the sequence, and by comparing it with the parent strain CJR100 (SEQ ID NO: 22 to SEQ ID NO: 28), variations that occurred in seven types of promoter regions were identified, and the sequences of the mutant promoters containing the said mutations are as shown in Table 4 below.

**[Table 4]**

| SEQ ID NO | Name | Expressed protein (regulated protein) | Promoter sequence (5'->3') |
|---|---|---|---|
| 15 | PBBD29_12 015 | hypothetical protein | CCTAAATCCAGCGCGTCTGCCAATTCCGCCACAAGCGCTT AATGCGGGTTTTACTATACATGGTGTAGTCCCAAATTAAC TAATGCGGGTGACCAACTGCACCGTAAAAAAAATTCGCT TATCGTAAATCAGCAGGTAGAATAACACTCT |
| 16 | PBBD29_07 560 | Argininosuccinate synthase | GGCTTGTTAATCGCTTGTTAATGCAGGCAGGTAAGGTATA ACCCGAGTGTTTTTTCGGGGAATACCAACCCTTTCAACAC AATAATTTTCTTTAAACATCCTTGCTGTCCACCACGGCTG GCAAGGAACTTAAAATGAAGGAGCACACCTC |
| 17 | PBBD29_12 275 | ribonucleotide-diphosphate reductase subunit beta | TAGGTGCGCTCTTCCCTTCTACAAATTTTTTTGCTTTTCG ACGCCACAATGCAGCGCGATTTCAGATCGCCAGCTTACC CCAACCGAAGCCCTTCAGAAGACCCGCCCCAGCCTATCA ATTTTGGTCAAGTCGCCTAGCTTTAGACCTAG |
| 18 | PBBD29_01 740 | phosphoserine phosphatase | AAGGGACGGCCCGCGCCTCGGGGGGGTGTGCGCGGGCCG TCAGTAACCCGGCAACGGGGGGGGGGATTGTGCCGGGGC AGGCCACACTGTATATCGGGGCCTTGCACCTGAAATTATG GCATATCAAACGCCACAAGACAACATCAAACCCGATTAT TTATTCGAATAACACGAATACTTAACAGTGTGGCAAATG |
| | | | ACACTTCCCGCACGCCTTCTACATTCCCAATGCGCGACCC CAGCACGTCACTTCAATATCATTGGTAACTTTAGTTTCTTT CTCAGTCTTGGAAAGTTGCCAAAAAGCGCTAAACTATGC GGTG |
| 19 | PBBD29_04 965 | D-lactate dehydrogenase | AGGAGGGGCCTACCCGGGCAGCGCCACGCCGTTCGATCC TTGACTACGCGCTACACCCTCATCTGTGAAGAGCCACCAA ACCTGGGACGGTTCAAGCGGCGCTAGGCGAGTCTGATTG CTGCGTCGATCTATGGGGTGAGGGCAGTGGCCGACATCG CGGTCCGAACTATCGCGGTCCGAACCTACCGGAGTCCGA AAGTACCGCGGTCCGAACCTACCGGAGTCCGAAAGTACC GCGGTCCGAACCTACCGGAGTCCGAAAGTACCGCGGTCC GAAACTATCTGATGCGCATCTTGTACAATGAGGATG |
| 20 | PBBD29_05 235 | BBD29_05235 | CGTCTCGAGGTGTCTTATATATAGAGGTATAGGGGAGAA AAATTGAGGGGGAGAATCCGGTCCCATCGACTTCCCCAT CACTGCAGTCGCCAAAAGAGTCTGTGAGTCACATCCCAG GATGATCTCATGGCAGGAAAAGTCGGCCCGGGAAAATAC CCCTGATCTTCATCGTGCCAGAGAAGGGCCGCGCATCTCG CGAGTGCGGCAGTATCCCTAAAAGTTACCCCCCCCCGAG ACTATAAGTTCGTGTAACTGGTGCTGTTGCAAAGTTGGGG CAGTAGGAAGACCGGCGTGAAATAATCAGG |
| 21 | PBBD29_14 250 | hypothetical protein | TGCGGTTGTCATGGTGACCTCTTCTCTGAAACGGACGGCG GGAGTAAACGGGCTGAAACCGATTGGCCCAACCTCATGG TCAGACCACAAGAATGTAAGGCATGTCACCTTTGATTGTC AATGGGGTGTAGCAAACAGGGGGTGGATTGTGTTGCCCT CGCCTTAACGTGTCTCCTGGGGTGTGGGGCTGGGTGACGT ATCTGAGACGCTCGGCCTTGGGGGGGGGAAGGTACGAAT ATAATTTCGAATAATGTATGATCAAGACTCGTCTTTAAAA TGGTATTTCAAATGTGAGGCAGT |

| | | | |
|---|---|---|---|
| (Expressed protein: a protein encoded by a gene whose expression is regulated by being operably linked to the promoter) | | | |

In the following examples, the effect of each mutant promoter listed in Table 4 on the L-arginine productivity of the genus *Corynebacterium* microorganism was evaluated to identify effective factors affecting the L-arginine production ability.

### EXAMPLE 4: Construction of an L-arginine-producing strain introduced with a mutant promoter

### Example 4-1: Construction of a recombinant vector for introducing a mutant promoter

In order to insert each of the mutant promoters of PBBD29_12015, PBBD29_07560, PBBD29_12275, BBD29_01740, PBBD29_04965, PBBD29_05235 and PBBD29_14250 in Table 4 into CJR100 strain, a vector comprising the target mutation was constructed.

Specifically, the genomic DNA of the CJR100_mt3 strain was extracted using a G-spin Total DNA Extraction Mini Kit (Intron, Cat. No. 17045) according to the protocol provided in the kit, and PCR was performed using the genomic DNA as a template. SolgTM Pfu-X DNA polymerase was used as the polymerase, and the PCR conditions were as follows: denaturation at 95°C for 4 minutes; 27 cycles of denaturation at 95°C for 30 seconds, annealing at 60°C for 30 seconds, and polymerization at 72°C for 50 seconds; and polymerization was performed at 72°C for 5 minutes. The sequences of the primer pairs used in the experiment are shown in Table 5 below.

**[Table 5]**

| Primer name | Sequence (5'->3') | SEQ ID NO |
|---|---|---|
| PBBD29_12015*_F | tgaattcgagctcggtacccGTGATTCGTATTCCTCGGTG | 29 |
| PBBD29_12015*_R | gtcgactctagaggatccccGTCTTCTTGCTCTGGCTGCG | 30 |
| PBBD29_07560*_F | tgaattcgagctcggtacccGTTGTCATCACCGATACCTG | 31 |
| PBBD29_07560*_R | gtcgactctagaggatccccGCTTCATGTACATGCCGTTT | 32 |
| PBBD29_12275*_F | tgaattcgagctcggtacccAGCACCGCTTCTTCGTGCAG | 33 |
| PBBD29_12275*_R | gtcgactctagaggatccccAGTTTCCCACCTTTTTGAAG | 34 |
| PBBD29_01740*_F | tgaattcgagctcggtacccCGATGATGGTCTTGTCCAGA | 35 |
| PBBD29_01740*_R | gtcgactctagaggatccccTGCCTGGAGATGTCCATCAA | 36 |
| PBBD29_04965*_F | tgaattcgagctcggtacccCCACCAGTCAGGCCCGTGTT | 37 |
| PBBD29_04965*_R | gtcgactctagaggatccccTACTCAGGGATATCTTTCGA | 38 |
| PBBD29_05235*_F | tgaattcgagctcggtacccTCGCCGCGTTGTGTAAAGAA | 39 |
| PBBD29_05235*_R | gtcgactctagaggatccccAGGTAGCACCACCTGCCGCC | 40 |
| PBBD29_14250*_F | tgaattcgagctcggtacccTTCTAGATCTATTCGCCGAT | 41 |
| PBBD29_14250*_R | gtcgactctagaggatccccTTGAAGACCAGCGCGGCGAC | 42 |

The above-obtained mutation introduction fragment and the pDC24 vector (SEQ ID NO: 43) treated with the restriction enzyme SmaI were cloned using the Gibson assembly method (DG Gibson et al., NATURE METHODS, VOL.6 NO.5, MAY 2009, NEBuilder HiFi DNA Assembly Master Mix) to obtain recombinant plasmids, and the vectors pDC24-Pn*_BBD29_12015; pDC24-Pn*_BBD29_07560; pDC24-Pn*_BBD29_12275; pDC24-Pn*_BBD29_01740; pDC24-Pn*_BBD29_04965; pDC24-Pn*_BBD29_05235; and named pDC24-Pn*_BBD29_14250.

### EXAMPLE 4-2: Construction of an L-arginine-producing strain introduced with a mutant promoter

The 7 types of vectors prepared in the above Example 4-1 were transformed into the CJR100 strain by an electroporation method, and strains in which the vector was inserted onto a chromosome by recombination of a homologous sequence were selected using a kanamycin medium. Thereafter, the strain into which the mutant promoter was introduced was confirmed through PCR using primers of SEQ ID NO: 44 and SEQ ID NO: 45; or SEQ ID NO: 46 and SEQ ID NO: 47; or SEQ ID NO: 48 and SEQ ID NO: 49; or SEQ ID NO: 50 and SEQ ID NO: 51; or SEQ ID NO: 52 and SEQ ID NO: 53; or SEQ ID NO: 54 and SEQ ID NO: 55; or SEQ ID NO: 56 and SEQ ID NO: 57; for the transformant strain in which the secondary recombination was completed. PCR was performed in the same manner as Example 4-1. The recombinant strains were named CJR100△Pn_BBD29_12015::Pn*_BBD29_12015, CJR100△Pn_BBD29_07560::Pn*_BBD29_07560, CJR100△Pn_BBD29_12275::Pn*_BBD29_12275, CJR100△Pn_BBD29_01740::Pn*_BBD29_01740, CJR100△Pn_BBD29_04965::Pn*_BBD29_04965, CJR100△Pn_BBD29_05235::Pn*_BBD29_05235, and CJR100△Pn_BBD29_14250::Pn*_BBD29_14250, respectively. The sequences of the primer pairs used for confirmation are shown in Table 6 below.

**[Table 6]**

| SEQ ID NO | Name | Sequence (5'->3') |
|---|---|---|
| 44 | PBBD29_12015*_CF | GGTGATCCCAGCGATACT |
| 45 | PBBD29_12015*_CR | ATAGGGCTTCAGCAAAGC |
| 46 | PBBD29_07560*_CF | AGGGTAAGAAGGCTGTGT |
| 47 | PBBD29_07560*_CR | GGCGATAGCCTTGTCGCG |
| 48 | PBBD29_12275*_CF | ACAGGAAGGACTCCAGGA |
| 49 | PBBD29_12275*_CR | CTCTGTGCCTTCATCCAG |
| 50 | PBBD29_01740*_CF | GGGTGTTGTCCATTTGTT |
| 51 | PBBD29_01740*_CR | TTGCTCAGCGTGGATTTT |
| 52 | PBBD29_04965*_CF | AGATGATCACAATGGGGC |
| 53 | PBBD29_04965*_CR | GGTTGAAGATCTGTCGGG |
| 54 | PBBD29_05235*_CF | CGTCCGCTGCAAATATCT |
| 55 | PBBD29_05235*_CR | TATAACGTTGTCCGGGCT |
| 56 | PBBD29_14250*_CF | ATTCTTCATCTTCGACGG |
| 57 | PBBD29_14250*_CR | TCAAAGTGATGAACAGGG |

### EXMPLE 5. Evaluation of L-arginine productivity of the L-arginine-producing strain introduced with a mutant promoter

To evaluate the L-arginine production ability of the recombinant strains constructed in Example 4-2, they were cultured and evaluated using the following method.

Each strain was inoculated into a 250-ml corner-baffle flask containing 25 ml of seed medium and cultured at 30°C for 20 hours with shaking at 200 rpm. One ml of the seed culture was inoculated into a 250-ml corner-baffle flask containing 24 ml of production medium and cultured at 30°C for 54 hours with shaking at 200 rpm. The medium composition was the same as in Example 2-2, and the experiment was repeated three times.

After the culture was completed, the amount of L-arginine produced was measured using high-performance liquid chromatography (HPLC), and the average value of the analysis results is shown in Table 7 below.

**[Table 7]**

| Strain Name | L-arginine concentration(g/L) |
|---|---|
| CJR100 | 5.9 |
| CJR100△Pn_BBD29_12015::Pn*_BBD29_12015 | 6.05 |
| CJR100△Pn_BBD29_07560::Pn*_BBD29_07560 | 6.03 |
| CJR100△Pn_BBD29_12275::Pn*_BBD29_12275 | 6.1 |
| CJR100△Pn_BBD29_01740::Pn*_BBD29_01740 | 6.08 |
| CJR100△Pn_BBD29_04965::Pn*_BBD29_04965 | 6.01 |
| CJR100△Pn _BBD29_05235::Pn*_BBD29_05235 | 5.99 |
| CJR100△Pn _BBD29_14250::Pn*_BBD29_14250 | 6.24 |

As shown in Table 7, the arginine production of strains introduced with mutant promoters either increased or reached levels comparable to those of the parent strain. In particular, "CJR100△Pn_BBD29_14250::Pn*_BBD29_14250" showed significantly improved L-arginine production compared to the parent strain CJR100.

This confirms that introducing a mutant promoter that controls the expression of the BBD29_14250 gene can result in more efficient L-arginine production.

From the above description, those skilled in the art will understand that the present disclosure can be implemented in other specific forms without altering its technical concept or essential characteristics. In this regard, it should be understood that the embodiments described above are illustrative in all respects and not restrictive. The scope of this disclosure should be interpreted to include all changes or modifications derived from the meaning and scope of the following claims and their equivalents, rather than the detailed description above.

## Claims

1. A polynucleotide comprising a nucleotide sequence having at least 90% sequence identity with the nucleotide sequence of SEQ ID NO: 21.

2. The polynucleotide of claim 1, wherein the polynucleotide comprises the nucleotide sequence of SEQ ID NO: 21.

3. An expression cassette comprising the polynucleotide of claim 1 and a target gene.

4. A microorganism of the genus *Corynebacterium* comprising the polynucleotide of claim 1 or claim 2; or the polynucleotide and a target gene operably linked thereto.

5. The microorganism of claim 4, wherein the microorganism of the genus *Corynebacterium* is *Corynebacterium glutamicum.*

6. A method for producing L-arginine, comprising culturing the microorganism of claim 4 in a medium.

7. The method for producing L-arginine of claim 6, further comprising recovering L-arginine from the cultured medium or microorganism.

8. A use of the microorganism of claim 4 or claim 5, for producing L-arginine.

9. A composition, method, product, process, or use **characterized by** one or more elements disclosed herein.
